(19) **Europäisches Patentamt / European Patent Office / Office européen des brevets**

(11) **EP 1 832 313 A1**

(12) **EUROPEAN PATENT APPLICATION**

(43) Date of publication:
**12.09.2007 Bulletin 2007/37**

(51) Int Cl.:
**A61N 5/10** (2006.01)

(21) Application number: **07102488.9**

(22) Date of filing: **15.02.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **10.03.2006 JP 2006065708**

(71) Applicant: **Mitsubishi Heavy Industries, Ltd.
Minato-ku
Tokyo (JP)**

(72) Inventors:
• **Urano, Susumu**
 **Hiroshima, Hiroshima-ken (JP)**
• **Kaneko, Shuji**
 **Hiroshima, Hiroshima-Ken (JP)**
• **Kawada, Noriyuki**
 **Hiroshima, Hiroshima-Ken (JP)**

(74) Representative: **Intes, Didier Gérard André et al
Cabinet Beau de Loménie,
158, rue de l'Université
75340 Paris Cedex 07 (FR)**

(54) **Radiotherapy device control apparatus and radiation irradiation method**

(57) A radiotherapy device control apparatus controlling a radiotherapy device (3), includes an affected area position database (62), a motion collection section (61), and an irradiation position control section (58). The radiotherapy device (3) includes a therapeutic radiation irradiation device (16) radiating therapeutic radiation, a motion detector (4) detecting motion of the subject, and a drive device (11, 15, 42) moving the therapeutic radiation irradiation device (16) with respect to the subject. The irradiation position control section (58) moves the therapeutic radiation irradiation device (16) by the drive device (11, 15, 42) such that the therapeutic radiation is radiated to a position in the position set corresponding to motion related information of the motion.

Fig. 2

EP 1 832 313 A1

## Description

## Background of the Invention

### 1. Field of the Invention

The present invention relates to a radiotherapy device control apparatus and a radiation irradiation method, and more specifically to a radiotherapy device control apparatus and a radiation irradiation method for use in treating a patient by irradiating his or her affected area with radiation.

### 2. Description of the Related Art

Radiotherapy is known which treats a patient by irradiating his or her affected area (tumor) with radiation. It is desired that the radiotherapy provides a high therapeutic effect and that a dosage of radiation thereof irradiated to normal cells be smaller than a dosage of radiation irradiated to cells at the affected area.

The radiotherapy device is known which tracks a position of an affected area based on transmitted images photographed by applying diagnostic X-rays, and irradiates the position with a radiation for treatment. It is desired that a dosage of the diagnostic X-rays to a patient be as small as possible.

Japanese patent JP37467478 discloses a radiotherapy device capable of monitoring, in real time, condition of a treatment field even during radiation irradiation treatment. The radiotherapy device includes: a radiation irradiation head which irradiates a treatment field of a subject with therapeutic radiation; an X-ray source which irradiates the treatment field of the subject with diagnostic X-rays; a sensor array which detects transmitted X-rays of the diagnostic X-rays transmitted through the subject and then outputs them as diagnostic image data. The sensor array moves in conjunction with movement of the radiation irradiation head.

Japanese Laid Open Patent Application JP2004-121406A discloses a radiotherapy device capable of easily positioning, within a radiation irradiation range, a target to be irradiated with radiation. This radiotherapy device is characterized by being provided with: a radiation generator which emits radiation and laser beams concentrically with each other; a guide which moves the radiation generator along an orbit of a predetermined radius with respect to an isocenter serving as a center so that irradiation axes of the radiation and the laser beams emitted concentrically with each other intersect with each other at one point; a support member which rotates the guide around an inclined axis passing through the isocenter; a movable member which pivots the radiation generator by rotating axes intersecting with each other and moves along the guide; a detector which detects information of a transmitted image in a range including the isocenter and the target to be irradiated with the radiation arranged near the isocenter; an analyzer which calculates relative positional relationship between the isocenter and the target to be irradiated based on information of a plurality of the transmitted images respectively detected in a plurality of orientations by the detector and based on information of the orientations in which the transmitted images are detected with respect to the isocenter; and a controller which moves the radiation generator based on the relative positional relationship.

Japanese patent JP3053389B (corresponding to US6307914B) discloses a moving body tracing irradiation device capable of automatically calculating, in actual time, position of tumor moving around in the trunk and capable ensuring the actually required accuracy without depending on the absolute accuracy of a mechanical system. This moving body tracing irradiation device is characterized by being provided with: a linac which irradiates tumor with therapeutic beams; a tumor marker which is embedded near the tumor; a first x-ray fluoroscope which images the tumor marker from a first direction; a second x-ray fluoroscope which images the tumor marker from a second direction simultaneously with the first x-ray fluoroscope; a first and a second image input parts which digitize a first and a second fluoroscopic images outputted from the first and second x-ray fluoroscopes; a first and a second recognition processing parts which execute, at an actual time level of a predetermined frame rate, template matching by a gray scale normalization cross-correlation method in which a template image of a tumor marker previously registered is effected on image information digitized by the first and second image input parts to thereby obtain first and second two-dimensional coordinates of the tumor marker; a central processing part which calculates three-dimensional coordinates of the tumor marker from the first and second two-dimensional coordinates calculated by the first and second recognition processing parts; and an irradiation control part which controls the therapeutic beams of the linac based on the obtained three-dimensional coordinates of the tumor marker.

Japanese Patent JP3432268B discloses a radiotherapy system capable of resolving deterioration in accuracy in irradiation planning due to body movement of a subject during radiotherapy. The radiotherapy system is characterized by being provided with: in radiotherapy system performing radiotherapy by irradiating a subject with radiation, phase identification means adapted to establish association between the phase of living body data in accordance with the body movement of the subject detected in parallel with acquisition of a CT image of the subject and the phase of the CT image; plan data creation means adapted to, based on the CT image associated with the phase of the living body data by the phase identification means, create treatment plan data including opening and closing data of a collimator for adjusting a field to be irradiated with the radiation; determination means adapted to determine correlation between the living body data associated with the phase of the CT image and the living body data in accordance with the body movement of the subj ect obtained during the radiotherapy; and collimator control means adapted to, in accordance with a result of determination made by the determination means, perform opening and closing control of the collimator based on the opening and closing data.

Japanese Patent JP3326597B discloses a respiration synchronizing control device. This control device is characterized by the use of function of a semiconductor position detecting element (PSD) and composed of: a light source part with alight source position or alight direction fluctuating in correspondence with the fluctuation of the outer skin of an organism interlocked with respiration; the PSD receiving light from the light source part, as a fluctuation signal of the outer skin of the organism and converting it into an electric signal corresponding to the cycle phase of respiration; and a control circuit sending an actuation control signal of other controlled equipment on the basis of this electric signal.

Japanese Laid Open Patent Application JP H4-507048A (corresponding to W09011721) discloses a patient alignment system and procedure for radiation treatment. In this patent, accurate and repeatable patient alignment with a charged-particle beam of a radiation beam therapy system, such as a proton beam delivery system, is provided. The patient is immobilized within a formfit patient pod. Reference radiographs are prepared with an X-ray system that is used for repositioning the patient within the pod on subsequent occasions. CT scan data is obtained using a CT Scan System of a particular tissue volume of interest, such as a region of the patient wherein a cancerous tumor is located, while the patient remains in the pod. The CT scan data is used to prepare a treatment plan for the patient . The treatment plan includes identifying an isocenter within the tissue volume at which the beam is to be directed from a selected angle (s). A computer simulation of the treatment plan is performed to optimize the treatment plan.

Japanese Patent JP3394250B (corresponding to W092006644) discloses an apparatus for and method of stereotaxic surgery. The method and the apparatus are set forth for selectively irradiating a target within a patient. A 3-dimensional mapping is provided of a mapping region surrounding the target. A beaming apparatus emits a collimated beam. Diagnostic beams at a known non-zero angle to one another pass through the mapping region. They produce images of projections within the mapping region. Electronic representations of the images are compared with the reference data thereby locating the target. The relative positions of the beaming apparatus and the living organism are adjusted in such a manner that the collimated beam is focused on the target region. The comparison is repeated at small time intervals and, when the comparison so indicates, the adjusting step is repeated, as needed, and in such a manner that the collimated beam remains focused onto the target region.

Japanese Laid Open Patent Application JP H8-511452A (corresponding to W09428971) discloses a radiotherapy system. The radiation therapy machine having constrained angular freedom to produce a beam only within a gantry plane. A radiation shield may be stationary and not attached to the gantry or rotating to always block the primary beam. The constrained motion reduces the risk of patient/gantry collision and provides for extremely accurate radiation therapy planning. The therapy machine, so constrained, may include a tomographic imaging system on a single gantry. The two systems cooperate and employ many of the same hardware components to both plan and carry out therapy sessions in which irregularly shaped treatment volumes are accurately irradiated while tissue surrounding those volumes is minimally irradiated. The therapy machine also may include a collimator that changes the width of a fan beam of radiation as a treatment volume of the patient crosses the volume exposed by the beam so as to minimize the irradiation of healthy tissue at the front and back of the tumor. The width of the fan beam may be controlled to treat multiple adjacent, similar slices of the patient at one time reducing the treatment duration.

**Summary of the Invention**

It is an object of the present invention to provide a radiotherapy device control apparatus and a radiation irradiation method which can certainly irradiate a part of a moving subject with radiation.

It is still another object of the present invention to provide a radiotherapy device control apparatus and a radiation irradiation method which can certainly irradiate a part of a moving subject, which is difficult to be photographed in a transmitted image, with radiation.

It is still another object of the present invention to provide a radiotherapy device control apparatus and a radiation irradiation method which can certainly irradiate a part of a moving subj ect with radiation, and further reduce a dosage of radiation irradiated to the subject.

It is still another object of the present invention to provide a radiotherapy device control apparatus and a radiation irradiation method which can certainly irradiate a part of a moving subject, which is difficult to be photographed in a transmitted image, with radiation, and further reduce a dosage of radiation irradiated to the subject.

It is still another object of the present invention to provide a radiotherapy device control apparatus and a radiation irradiation method in which a size of a radiotherapy device irradiating a part of a moving subject with radiation can be smaller.

It is still another object of the present invention to provide a radiotherapy device control apparatus and a radiation irradiation method in which a size of a radiotherapy system irradiating a part of a moving subject with radiation can be smaller.

This and other objects, features and advantages of the present invention will be readily ascertained by referring to the following description and drawings.

In order to achieve an aspect of the present invention, the present invention provides a radiotherapy device control apparatus including: an affected area position database; a motion collection section; and an irradiation position control section. The radiotherapy device control apparatus controls a radiotherapy device. The radiotherapy device includes: a therapeutic radiation irradiation device which irradiates a part of a subject with therapeutic radiation, a motion detector which detects motion of the subject, and a drive device which moves the therapeutic radiation irradiation device with respect to the subject. The affected area position database associates a position set with a motion related information set related to the motion. The motion collection section collects the motion from the motion detector. The irradiation position control section moves the therapeutic radiation irradiation device by the drive device such that the therapeutic radiation is radiated to a position in the position set corresponding to motion related information of the motion.

In the radiotherapy device control apparatus, the motion related information set may be a motion set, and the motion related information may be the motion.

In the radiotherapy device control apparatus, the motion may indicate a landmark position at which a landmark arranged in the subject is displayed in a transmitted image taken by an imager of the radiotherapy device using radiation transmitted through the subject.

In the radiotherapy device control apparatus, the affected area position database may further associate a set of an average rate of change with the position set. The irradiation position control section may move the therapeutic radiation irradiation device by the drive device such that the therapeutic radiation is radiated to a position in the position set further corresponding to an average rate of change of the motion.

In the radiotherapy device control apparatus, the irradiation position control section may make the therapeutic radiation irradiation device stop radiating the therapeutic radiation if the motion is not included in a predetermined range.

The radiotherapy device control apparatus may further include: an affected area position table creation section which creates the affected area position database based on variation of a three-dimensional data of the subject created by a three-dimensional imaging device provided in addit ion to the radiotherapy device and variation of a mot ion detected by the motion detector.

The radiotherapy device control apparatus may further include: an affected area position table creation section which creates the affected area position database based on variation of a three-dimensional data of the subject created by using a transmitted image taken by an imager of the radiotherapy device using transmitted radiation transmitted through the subject and variation of a motion detected by the motion detector.

In the radiotherapy device control apparatus, the transmitted radiation may be generated by the therapeutic radiation being transmitted through the subject.

In the radiotherapy device control apparatus, the motion related information set is a set of an average rate of change, and the motion related information is an average rate of change of the motion.

The present invention provides a radiotherapy device control apparatus including: a motion collection section; and a therapeutic radiation irradiation section. The radiotherapy device control apparatus controls a radiotherapy device. The radiotherapy device includes: a therapeutic radiation irradiation device which radiates therapeutic radiation, and a motion detector which detects motion of the subject. The motion collection section collects the motion from the motion detector. The therapeutic radiation irradiation section makes the therapeutic radiation irradiation device radiate the therapeutic radiation when the average rate of change is included in a predetermined range, and makes the therapeutic radiation irradiation device stop radiating the therapeutic radiation when the average rate of change is not included in the predetermined range.

In order to achieve another aspect of the present invention, the present invention provides a radiotherapy system including: a radiotherapy device control apparatus according to any of the above mentioned radiotherapy device control apparatuses; and a radiotherapy device.

In order to achieve another aspect of the present invention, the present invention provides a radiation irradiation method using a radiotherapy device. The radiotherapy device includes: a therapeutic radiation irradiation device which irradiates a part of a subject with therapeutic radiation, a motion detector which detects motion of the subject, and a drive device which moves the therapeutic radiation irradiation device with respect to the subject. The radiation irradiation method includes:

> (a) collecting the motion from the motion detector; and
> (b) moving the therapeutic radiation irradiation device by the drive device with reference to an affected area position database associating a position set with a motion related information set related to the motion, such that the therapeutic radiation is radiated to a position in the position set corresponding to motion related information of the motion.

In the radiation irradiation method, the motion related information set may be a motion set, and the motion related information may be the motion.

In the radiation irradiation method, the motion may indicate a landmark position at which a landmark arranged in the subject is displayed in a transmitted image taken by an imager of the radiotherapy device using radiation transmitted through the subject.

In the radiation irradiation method, the affected area position database may further associate a set of an average rate of change with the position set. The position may further correspond to an average rate of change of the motion.

The radiation irradiation method may further include:

(c) making the therapeutic radiation irradiation device stop radiating the therapeutic radiation if the motion is not included in a predetermined range.

The radiation irradiation method may further include:

(e) creating the affected area position database based on variation of a three-dimensional data of the subject created by a three-dimensional imaging device provided in addition to the radiotherapy device and variation of a motion detected by the motion detector.

The radiation irradiation method may further include:

(f) creating the affected area position database based on variation of a three-dimensional data of the subj ect created by using a transmitted image taken by an imager of the radiotherapy device using transmitted radiation transmitted through the subject and variation of a motion detected by the motion detector.

In the radiation irradiation method, the transmitted radiation may be generated by the therapeutic radiation being transmitted through the subject.

In the radiation irradiation method, the motion related information set may be a set of an average rate of change. The motion related information may be an average rate of change of the motion.

The present invention provides a radiation irradiation method using a radiotherapy device. The radiotherapy device includes: a therapeutic radiation irradiation device which radiates therapeutic radiation, and a motion detector which detects motion of the subject. The radiation irradiation method includes: (h) collecting the motion from the motion detector; (j) making the therapeutic radiation irradiation device radiate the therapeutic radiation when the average rate of change is included in a predetermined range; and (k) making the therapeutic radiation irradiation device stop radiating the therapeutic radiation when the average rate of change is not included in the predetermined range.

In order to achieve another aspect of the present invention, the present invention provides computer program product with program code means for carrying out all steps according to any of the above-mentioned radiation irradiation methods if the program runs on a computer.

The present invention provides computer program product with program code means according to the above-mentioned computer program product which are stored on a storage means which can be read by the computer.

A radiotherapy device control apparatus and a radiation irradiation method according to the present invention can certainly irradiate a part of a moving subj ect with radiation.

The radiotherapy device control apparatus and a radiation irradiation method according to the present invention can certainly irradiate a part of a moving subject, which is difficult to be photographed in a transmitted image, with radiation.

The radiotherapy device control apparatus and a radiation irradiation method according to the present invention can certainly irradiate a part of a moving subject with radiation, and further reduce a dosage of radiation irradiated to the subject.

The radiotherapy device control apparatus and a radiation irradiation method according to the present invention can certainly irradiate a part of a moving subject, which is difficult to be photographed in a transmitted image, with radiation, and further reduce a dosage of radiation irradiated to the subject.

In the radiotherapy device control apparatus and a radiation irradiation method according to the present invention, a size of a radiotherapy device irradiating a part of a moving subject with radiation can be smaller.

In the radiotherapy device control apparatus and a radiation irradiation method according to the present invention, a size of a radiotherapy system irradiating a part of a moving subject with radiation can be smaller.

**Brief Description of the Drawings**

Fig. 1 is a block diagram showing a radiotherapy system in the embodiment of the present invention;
Fig. 2 is a perspective view showing a radiotherapy device of the radiotherapy system in the embodiment of the present invention;
Fig. 3 is a block diagram showing a radiotherapy device control apparatus of the radiotherapy system in the embodiment of the present invention;
Fig. 4 is a view showing an affected area position table stored in a storage device in the embodiment of the present invention;
Fig. 5 is a view showing a permitted range table in the embodiment of the present invention;
Fig. 6 is a graph showing changes in a respiration rate measured by a respirometer; and
Fig. 7 is a view showing a screen displayed in an output device in the embodiment of the present invention.

**Description of the Preferred Embodiments**

The invention will be now described herein with reference to illustrative embodiments. Those skilled in the art will recognize that many alternative embodiments can be accomplished using the teachings of the present invention and that the invention is not limited to the embodiments illustrated for explanatory purposed.

Embodiments of a radiotherapy system according to the present invention will be described below referring to the accompanying drawings.

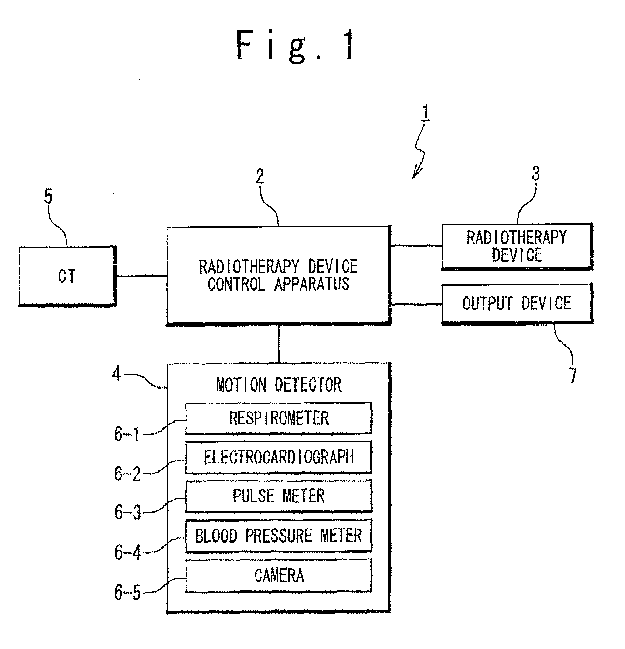

Fig. 1 is a block diagram showing a radiotherapy system 1. The radiotherapy system 1 is provided with a radiotherapy device control apparatus 2, a radiotherapy device 3, a motion detector 4, a CT (computerized tomographic apparatus) 5, and an output device 7. The radiotherapy device control apparatus 2 is a computer exemplified by a personal computer. The radiotherapy device control apparatus 2 is connected to the radiotherapy device 3 so as to be capable of transmitting information bi-directionally and also connected to the motion detector 4, the CT 5, and the output device 7 so as to be capable of transmitting information bi-directionally.

The motion detector 4 is composed of an apparatus which detects the motion of the human body without using radiation transmitted through the human body. The apparatus is composed of a respirometer 6-1, an electrocardiograph 6-2, a pulse meter 6-3, a blood pressure meter 6-4, and a camera 6-5. The respirometer 6-1 measures the respiration rate of a patient. The respiration rate indicates the rate of change caused by respiration of the patient, for example, the amount of air accumulated in the patient's lung. The electrocardiograph 6-2 creates an electrocardiogram of the patient and measures the activity rate of the patient's heart. The pulse meter 6-3 measures the pulse of the patient. The blood pressure meter 6-4 measures the blood pressure of the patient. The camera 6-5 forms a picture of the patient.

The CT 5 photographs (takes) a plurality of transmitted images by transmitting X-rays through a human body from various directions, and then subjects the plurality of transmitted images to image processing by a computer to thereby generate images of cross sections of the human body and also subjects the plurality of transmitted images to image processing by the computer to thereby generate three-dimensional data indicating inner condition of the human body. The CT 5 can be replaced with a different device, for example, an MRI device, which measures three-dimensional condition of the human body. The MRI device detects magnetism possessed by cells in the human body by using nuclear magnetic resonance and then transforms this magnetism into an image by a computer to thereby generate three-dimensional data indicating inner condition of the human body.

The output device 7 is a display arranged at position visible to the patient, and displays a screen generated by the radiotherapy device control apparatus 2 in a manner such that the patient can recognize the screen.

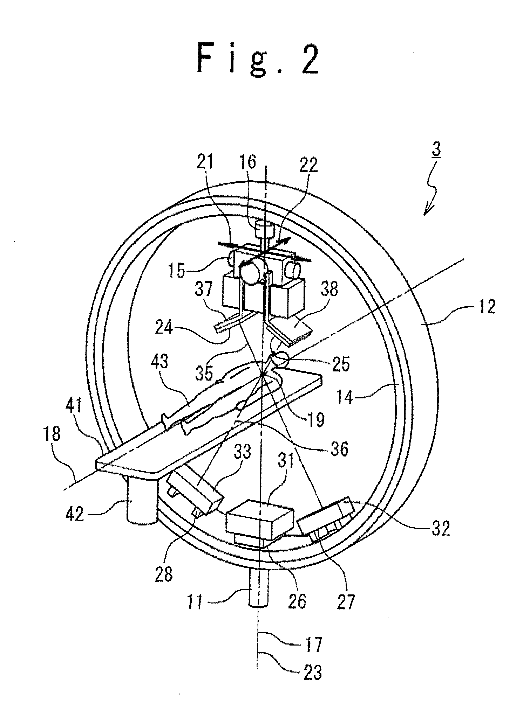

Fig. 2 is a perspective view showing the radiotherapy device 3 of the radiotherapy system 1 in the embodiment. The radiotherapy device 3 is provided with a turning drive device 11, an O ring 12, a travel gantry 14, a head swing device 15, and a therapeutic radiation irradiation device 16. The turning drive device 11 supports the O ring 12 to a base so that the O ring 12 is rotatable around a rotation axis 17, and is controlled by the radiotherapy device control apparatus 2 to rotate the O ring 12 around the rotation axis 17. The rotation axis 17 is parallel with the vertical direction. The O ring 12 is formed into a ring shape with a rotation axis 18 serving as a center, and supports the travel gantry 14 so that the travel gantry 14 is rotatable around the rotation axis 18. The rotation axis 18 is perpendicular to the vertical direction, and passes through an isocenter 19 included in the rotation axis 17. The rotation axis 18 is further fixed with respect to the O ring 12, that is, the O ring 12 rotates the travel gantry 14 around the rotation axis 18, and rotates round the rotation axis 18. The travel gantry 14 is formed into a ring shape with the rotation axis 18 serving as a center, and so arranged as to be concentric with the ring of the O ring 12. The radiotherapy device 3 is further provided with a traveling drive device, which is not shown. The traveling drive device (not shown) is controlled by the radiotherapy device control apparatus 2 to rotate the travel gantry 14 around the rotation axis 18.

The head swing device 15 is fixed inside the ring of the travel gantry 14 to support the therapeutic radiation irradiation device 16 to the travel gantry 14. The head swing device 15 has a pan axis 21 and a tilt axis 22. The pan axis 21 is fixed with respect to the travel gantry 14 and is parallel to the rotation axis 18 without intersecting therewith. The tilt axis 22 is fixed with respect to the travel gantry 14 and orthogonal to the pan axis 21. The head swing device 15 is controlled by the radiotherapy device control apparatus 2 to rotate the therapeutic radiation irradiation device 16 around the pan axis 21 and also rotates the therapeutic radiation irradiation device 16 around the tilt axis 22. The therapeutic radiation irradiation device 16 is controlled by the radiotherapy device control apparatus 2 to radiate therapeutic radiation 23.

Once the therapeutic radiation irradiation device 16 is supported by the travel gantry 14 as described above and is adjusted by the head swing device 15 so as to be directed toward the isocenter 19, the therapeutic radiation 23 always passes approximately through the isocenter 19 even when the O ring 12 is rotated by the turning drive device 11 or when the travel gantry 14 is rotated by the traveling drive device.

The radiotherapy device 3 is further provided with a plurality of imager systems. Specifically, the radiotherapy device 3 is provided with radiation source drive devices 37 and 38, diagnostic X-ray sources 24 and 25, sensor array drive devices 27 and 28, and sensor arrays 32 and 33. The radiation source drive device 37 is fixed inside the ring of the travel gantry 14, supports the_ diagnostic X-ray source 24 to the travel gantry 14, and is controlled by the radiotherapy device control apparatus 2 to move the diagnostic X-ray source 24 with respect to the travel gantry 14. The diagnostic X-ray source 24 is arranged inside the ring of the travel gantry 14 and at position such that a line segment linking from the isocenter 19 to the diagnostic X-ray source 24 and a line segment linking from the isocenter 19 to the therapeutic radiation irradiation device 16 forms an acute angle. The diagnostic X-ray source 24 is controlled by the radiotherapy device control apparatus 2 to radiate_diagnostic X-rays 35 toward the isocenter 19. The diagnostic X-rays 35 are radiated from one point included in the diagnostic X-ray source 24, and are cone beams of a conical shape with the aforementioned point serving as_a vertex. The radiation source drive device 38 is fixed inside the ring of the travel gantry 14, supports the diagnostic X-ray source 25 to the travel gantry 14, and is controlled by the radiotherapy device control apparatus 2 to move the diagnostic X-ray source 24 with respect to the travel gantry 14. The diagnostic X-ray source 25 is arranged inside the ring of the travel gantry 14 and at position such that a line segment linking from the isocenter 19 to the diagnostic X-ray source 25 and a line segment linking from the isocenter 19 to the therapeutic radiation irradiation device 16 forms an acute angle. The diagnostic X-ray source 25 is controlled by the radiotherapy device control apparatus 2 to radiate diagnostic X-rays 36 toward the isocenter 19. The diagnostic X-rays 36 are radiated from one point included in the diagnostic X-ray source 25, and are cone beams of a conical shape with the aforementioned point serving as a vertex.

The sensor array drive device 27 is fixed inside the ring of the travel gantry 14, supports the sensor array 32 to the travel gantry 14, and is controlled by the radiotherapy device control apparatus 2 to move the sensor array 32 with respect to the travel gantry 14. The sensor array drive device 28 is fixed inside the ring of the travel gantry 14, supports the sensor array 33 to the travel gantry 14, and is controlled by the radiotherapy device control apparatus 2 to move the sensor array 33 with respect to the travel gantry 14. The sensor array 32 receives the diagnostic X-rays 35 radiated by the diagnostic X-ray source 24 and transmitted through a subject around the isocenter 19 to generate a transmitted image of the subject. The sensor array 33 receives the diagnostic X-rays 36 radiated by the diagnostic X-ray source 25 and transmitted through the subject around the isocenter 19 to generate a transmitted image of the subject. The sensor arrays 32 and 33 are exemplified by FPDs (Flat Panel Detectors) and X-rays II (Image Intensifiers).

According to such imager systems, even when the_ diagnostic X-ray sources 24 and 25 are moved by the radiation source drive devices 37 and 38, the sensor arrays 32 and 33 can be appropriately moved by the sensor array drive devices 27 and 28, respectively, thus generating transmitted images with the isocenter 19 serving as a center thereof.

The radiotherapy device 3 is further provided with a sensor array drive device 26 and a sensor array 31. The sensor array drive device 26 is fixed inside the ring of the travel gantry 14, supports the sensor array 31 to the travel gantry 14, and is controlled by the radiotherapy device control apparatus 2 to move the sensor array 31 with respect to the travel gantry 14. The sensor array 31 receives the therapeutic radiation 23 radiated by the therapeutic radiation irradiation device 16 and transmitted through a subject around the isocenter 19 to generate a transmitted image of the subject. The sensor array 31 is exemplified by an FPD (Flat Panel Detector) and X-rays II (Image Intensifier). In this case, even when the therapeutic radiation irradiation device 16 is moved by the head swing device 15, the sensor array 31 can be appropriately moved by the sensor array drive device 26, thus generating a transmitted image with the isocenter 19 serving as a center thereof.

The diagnostic X-ray source 24 can also be arranged at a position such that a line segment linking from the isocenter 19 to the diagnostic X-ray source 24 and a line segment linking from the isocenter 19 to the therapeutic radiation irradiation device 16 forms an obtuse angle. That is, the sensor array 32 is arranged at position such that a line segment linking from the isocenter 19 to the sensor array 32 and a line segment linking from the isocenter 19 to the therapeutic radiation irradiation device 16 forms an acute angle. The diagnostic X-ray source 25 can also be arranged at position such that a line segment linking from the isocenter 19 to the diagnostic X-ray source 25 and a line segment linking from the isocenter 19 to the therapeutic radiation irradiation device 16 forms an obtuse angle. That is, the sensor array 33 is arranged at position such that a line segment linking from the isocenter 19 to the sensor array 33 and a line segment linking from the isocenter 19 to the therapeutic_radiation irradiation device 16 forms an acute angle. In this case, the sensor arrays 32 and 33 are less likely to be irradiated with the therapeutic radiation 23 radiated from the therapeutic radiation irradiation device 16, which is preferable.

The radiation source drive devices 37 and 38 can also support the diagnostic X-ray sources 24 and 25, respectively, to the therapeutic radiation irradiation device 16. In this case, even when the therapeutic radiation irradiation device 16 is moved by the head swing device 15, the relative position of the radiotherapy device 3 with respect to the therapeutic radiation irradiation device 16 is fixed, so that the radiotherapy device 3 can more easily control the position of the diagnostic X-ray sources 24 and 25, which is preferable.

The radiotherapy device 3 is further provided with a couch 41 and a couch drive device 42. The couch 41 is used for laying a patient 43 to be treated by the radiotherapy system 1. The couch 41 is provided with a fixing tool (not shown). This fixing tool fixes the patient to the couch 41 so that he or she does not move. The couch drive device 42 supports the couch 41 to the base and is controlled by the radiotherapy device control apparatus 2 to move the couch 41.

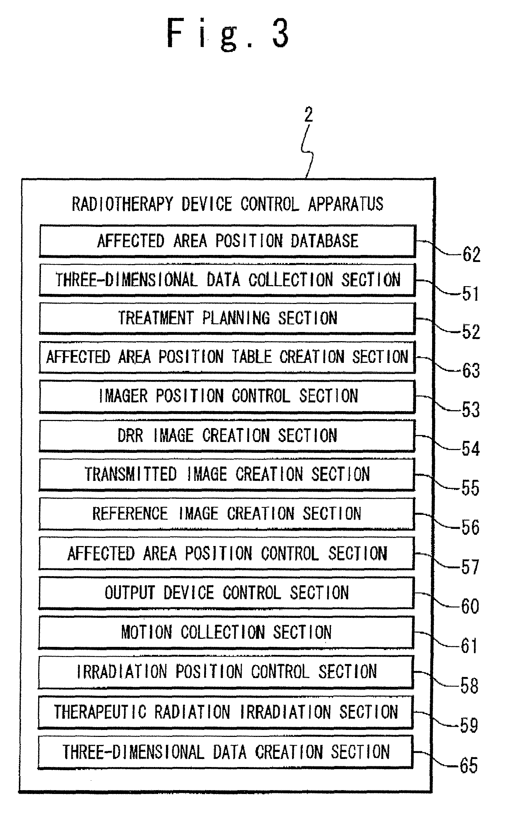

Fig. 3 is a block diagram showing the radiotherapy device control apparatus 2 of the radiotherapy system 1 in the embodiment. The radiotherapy device control apparatus 2 is a computer, and is provided with a CPU, a storage device, an input device, an output device, and an interface (all not shown). The CPU executes a computer program installed in the radiotherapy device control apparatus 2 to control the storage device, the input device, and the output device thereof. The storage device stores the computer program, information used by the CPU, and information generated by the CPU. The input device supplies to the CPU information generated through user's operation. The input device is exemplified by a keyboard and a mouse. The output device outputs information generated by the CPU in a manner such that the information can be recognized by the user. The output device is exemplified by a display. The interface outputs to the CPU information generated by an external device connected to the radiotherapy device control apparatus 2 and outputs to the external device information generated by the CPU.

The radiotherapy device control apparatus 2 is provided with: as computer programs, an affected area position database 62, a three-dimensional data collection section 51, a treatment planning section 52, an affected area position table creation section 63, an imager position control section 53, a DRR image creation section 54, a transmitted image creation section 55, a reference image creation section 56, an affected area position control section 57, an output device control section 60, a motion collection section 61, an irradiation position control section 58, and a therapeutic radiation irradiation section 59. Here, a three-dimensional data creation section 65 may be included in the radiotherapy device control apparatus 2.

The affected area position database 62 stores in the storage device an affected area position table indicating relationship between the motion of the patient and the affected area position in a manner such that the affected area position table can be searched and changed by the different computer programs.

The three-dimensional data collection section 51 collects from the CT 5 three-dimensional data generated by the CT 5 and indicating positional relationship between an affected area of the patient 43 and the organs around the affected area, and stores the three-dimensional data in the storage device in association with identification information of the patient 43. The three-dimensional data collection section 51 can also collect from the CT 5 a plurality of transmitted images photographed (taken) by transmitting X-rays through the patient 43 from various directions, can subject the plurality of transmitted images to image processing by a computer to thereby generate images of cross sections of the patient 43, and can subject the plurality of transmitted images to image processing by the computer to thereby generate three-dimensional data indicating inner condition of the patient 43.

The treatment planning section 52, based on the three-dimensional data collected by the three-dimensional data collection section 51 and information inputted by a user, creates a treatment plan, and stores the treatment plan in the storage device in association with the identification information of the patient 43. The treatment plan indicates irradiation angles at which the affected area of the patient 43 is irradiated with the therapeutic radiation 23 and the dosage and property of the therapeutic radiation 23 irradiated from each of the irradiation angles. The treatment plan further indicates imaging angles at which the diagnostic X-rays 35 and 36 are irradiated such that transmitted images taken by transmission of the diagnostic X-rays 35 and 36 through the patient 43 displays the affected area of the patient 43 more precisely when the therapeutic radiation 23 is irradiated from various irradiation angles. The imaging angles do not have to be indicated by the treatment plan, and can be inputted to the radiotherapy device control apparatus 2 separately from the treatment plan.

The imager position control section 53 controls the radiation source drive device 37 to move the diagnostic X-ray source 24 so that the diagnostic X-rays 35 are irradiated to the patient 43 at the imaging angle indicated by the treatment plan created by the treatment planning section 52, and controls the sensor array drive device 27 to move the sensor array 32 so that the transmitted image obtained by the diagnostic X-rays 35 mainly displays the affected area of the patient 43 in the center. The imager position control section 53 further controls the radiation source drive device 38 to move the diagnostic X-ray source 25 so that the diagnostic X-rays 36 are irradiated to the patient 43 at the imaging angle indicated by the treatment plan, and controls the sensor array drive device 28 to move the sensor array 33 so that the transmitted image obtained by the diagnostic X-rays 36 mainly displays the affected area of the patient 43 in the center. The imager position control section 53 further controls the radiation source drive device 38 to move the diagnostic X-ray source 25 so that the diagnostic X-rays 36 are irradiated to the patient 43 at the imaging angle, and controls the sensor array drive device 28 to move the sensor array 33 so that the transmitted image obtained by the diagnostic X-rays 36 mainly displays the affected area of the patient 43 in the center. The imager position control section 53 further controls the head swing device 15 to move the therapeutic radiation irradiation device 16 so that the therapeutic radiation 23 is irradiated to the patient 43 at the imaging angle indicated by the treatment plan, and controls the sensor array drive device 26 to move the sensor array 31 so that the transmitted image obtained by the therapeutic radiation 23 mainly displays the affected area of the patient 43 in the center.

The DRR image creation section 54, based on the_ three-dimensional data collected by the three-dimensional data collection section 51, calculates a DRR image, and stores the DRR image in the storage device in association with the identification information of the patient 43. The DRR image indicates a two-dimensional image which is taken when X-rays are transmitted at the imaging angle indicated by the treatment plan created by the treatment planning section 52. The DRR image creation section 54, based on information inputted by the user, further adds a mark to the position in the DRR image where a characteristic point of the patient 43 is displayed.

The transmitted image creation section 55 radiates the diagnostic X-rays 35 by using the diagnostic X-ray source 24 and takes a transmitted image of the patient 43 generated by using the sensor array 32 based on the diagnostic X-rays 35. The transmitted image creation section 55 further radiates the diagnostic X-rays 36 by using the diagnostic X-ray source 25 and takes a transmitted image of the patient 43 generated by using the sensor array 33 based on the diagnostic X-rays 36. The transmitted image creation section 55 further stores the transmitted images in the storage device in association with the identification information of the patient 43. With the plurality of transmitted images taken in this manner, three-dimensional position of the characteristic point of the patient 43 can be calculated. The characteristic point is exemplified by a portion (bone or the like) of internal organs of the patient 43 which is easy to appear in the transmitted image and a gold mark embedded in a portion thereof which moves in conjunction with the affected area of the patient 43. Further, the transmitted image creation section 55 can also radiate the therapeutic radiation 23 by using the therapeutic radiation irradiation device 16 and take a transmitted image of the patient 43 generated by using the sensor array 31 based on the therapeutic radiation 23. Further using the transmitted image obtained by the therapeutic radiation 23 permits calculating the three-dimensional position of the characteristic point of the patient 43 with higher accuracy. The transmitted image creation section 55 can also take only the transmitted image obtained by X-rays of one of the diagnostic X-rays 35 and 36 and the transmitted image obtained by the therapeutic radiation rays 23. Also in this case, the three-dimensional position of the characteristic point of the patient 43 can be calculated by using these two transmitted images. The transmitted image creation section 55 can also take only the transmitted image obtained by X-rays of one of the diagnostic X-rays 35 and 36 and the therapeutic radiation 23. In this case, the three-dimensional position of the characteristic point of the patient 43 cannot be calculated.

The reference image creation section 56 collects the transmitted images taken by the transmitted image creation section 55 from the storage device, and creates a reference image on the basis of the transmitted images. The transmitted images serving as a basis for creating the reference image is taken in the past before treatment operation executed by using this reference image, for example, a transmitted image taken by the transmitted image creation section 55 when the affected area of the patient 43 is so arranged as to be irradiated with the therapeutic radiation 23 in previous treatment operation. The reference image creation section 56, based on information inputted by the user, further adds a mark to position in the reference image where the characteristic point of the patient 43 is projected.

The affected area position control section 57 compares the transmitted image taken by the transmitted image creation section 55 with the DRR image generated by the DRR image creation section 54 to judge whether or not the affected area of the patient 43 is to be irradiated with the therapeutic radiation 23. The affected area position control section 57 further compares the transmitted image taken by the transmitted image creation section 55 with the DRR image generated by the DRR image creation section 54 to calculate such couch position of the couch 41 that permits the affected area of the patient 43 to be irradiated with the therapeutic radiation 23. For example, the couch position is position of the couch 41 when the affected area of the patient 43 is arranged at the isocenter 19. The couch position can be calculated based on the position of the mark added to the DRR image and the position of the characteristic point displayed in the transmitted image. The affected area position control section 57 moves the patient 43 by moving the couch 41 to the calculated position by using the couch drive device 42. That is, the affected area position control section 57 moves the patient 43 by using the couch drive device 42 so that the affected area of the patient 43 is irradiated with the therapeutic radiation 23. For example, the affected area position control section 57 moves the patient 43 by using the couch drive device 42 so that the transmitted image taken by the transmitted image creation section 55 matches with the DRR image generated by the DRR image creation section 54. For example, the affected area position control section 57 calculates a difference between the position of the characteristic point displayed in the transmitted image and the position of the characteristic point displayed in the DRR image. For example, the difference indicates the direction of the characteristic point of the DRR image with respect to the characteristic point of the transmitted image and the distance between the two characteristic points when the transmitted image and the DRR image are superimposed one on another. The affected area position control section 57, based on the difference, calculates the direction and distance in and by which the patient 43 is to be moved, and moves the couch 41 based on the direction and the distance.

The affected area position control section 57 further compares the transmitted image taken by the transmitted image creation section 55 with the reference image taken by the reference image creation section 56 to calculate such couch position of the couch 41 that permits the affected area of the patient 43 to be irradiated with the therapeutic radiation 23. The couch position can be calculated based on the position of the mark added to the reference image and the position of the characteristic point displayed in the transmitted image. For example, the affected area of the patient 43 is irradiated with the therapeutic radiation 23 if the relative position of the imager system with respect to the therapeutic radiation irradiation device 16 when the reference image is taken and the relative position of the imager system with respect to the therapeutic radiation irradiation device 16 when the transmitted image is taken match with each other and also if the position of the characteristic point in the transmitted image and the position of the characteristic point in the reference image match with each other. The affected area position control section 57 moves the patient 43 by moving the couch 41 to the calculated position by using the couch drive device 42. That is, the affected area position control section 57 moves the couch 41 so that the affected area of the patient 43 is irradiated with the_therapeutic radiation 23. For example, if the position of the imager system with respect to the therapeutic radiation irradiation device 16 when the reference image is taken and the position of the imager system with respect to the therapeutic radiation irradiation device 16 when the transmitted image is taken match with each other, the affected area position control section 57 moves the patient 43 by using the couch drive device 42 so that the transmitted image taken by the transmitted image creation section 55 matches with the reference image generated by the reference image creation section 56. For example, the affected area position control section 57 calculates a difference between the position of the characteristic point displayed in the transmitted image and the position of the characteristic point displayed in the reference image. For example, the difference indicates the direction of the characteristic point of the reference image with respect to the characteristic point of the transmitted image and the distance between the two characteristic points when the transmitted image and the reference image are superimposed one on another. The affected area position control section 57, based on the difference, calculates the direction and distance in and by which the patient 43 is to be moved, and moves the couch 41 based on the direction and the distance.

The affected area position control section 57 can also move the patient 43 by using the couch drive device_42 based on information inputted by the user. In this case, the affected area position control section 57 displays on the display the transmitted image taken by the transmitted image creation section 55 and the DRR image generated by the DRR image creation section 54. The user compares the transmitted image with the DRR image, and inputs to the radiotherapy device control apparatus 2 the direction and distance in and by which the patient 43 is moved. The affected area position control section 57 moves the patient 43 by using the couch drive device 42 based on the inputted information. Further, the affected area position control section 57 displays on the display the transmitted image taken by the transmitted image creation section 55 and the reference image generated by the reference image creation section 56. The user compares the transmitted image with the reference image, and inputs to the radiotherapy device control apparatus 2 the direction and distance in and by which the patient 43 is moved. The affected area position control section 57 moves the patient 43 by using the couch drive device 42 based on the inputted information.

The affected area position control section 57 can also control the relative position of the patient 43 with respect to the therapeutic radiation 23 by further using the turning drive device 11 or the traveling drive device for rotating the travel gantry 14 around the rotation axis 18 or the head swing device 15. In this case, the affected area position control section 57 uses the turning drive device 11 or the traveling drive device or the head swing device 15 preferentially prior to the couch drive device 42. Such movement can reduce the load of moving the patient 43, which is preferable.

The output device control section 60 displays on the output device 7 a screen indicating the respiration rate measured by the respirometer 6-1, instructing the patient 43 to perform predetermined respiration. The output device control section 60 can also display on the output device 7 a screen indicating the respiration rate requested to the patient 43, instructing the patient 43. Such instructions permit the respiration cycle of the patient 43 to be kept constant, thus reducing fluctuation in the respiration rate in each cycle of the respiration, which is preferable.

The motion collection section 61 collects from the motion detector 4 the value measured by the motion detector 4. Specifically, the motion collection section 61 collects from the respirometer 6-1 the patient's respiration rate measured by the respirometer 6-1, collects from the electrocardiograph 6-2 the activity rate of the patient's heart measured by the electrocardiograph 6-2, collects from the pulse meter 6-3 the pulse of the patient measured by the pulse meter 6-3, collects from the blood pressure meter 6-4 the patient's blood pressure measured by the blood pressure meter 6-4, and collects from the camera 6-5 a picture of the patient taken by the camera 6-5.

The affected area position table creation section 63, based on the values collected by the motion collection section 61 and the three-dimensional data collected by the three-dimensional data collection section 51, creates an affected area position table to be stored in the affected area position database 62.

The irradiation position control section 58, referring to the affected area position table stored in the storage device by the affected area position database 62, calculates the affected area position corresponding to the values collected by the motion collection section 61, and moves the therapeutic radiation irradiation device 16 by using the head swing device 15 so that the therapeutic radiation 23 is transmitted through the three-dimensional position. Further, the irradiation position control section 58 calculates three-dimensional position of the affected area based on the position of the affected area displayed in the transmitted image taken by the transmitted image creation section 55, and moves the therapeutic radiation irradiation device 16 by using the head swing device 15 so that the therapeutic radiation 23 is transmitted through the three-dimensional position. The irradiation position control section 58 can also move the therapeutic radiation irradiation device 16 by further using the turning drive device 11 or the traveling drive device for rotating the travel gantry 14 around the rotation axis 18 or the couch drive device 42 so that the therapeutic radiation 23 is transmitted through the three-dimensional position. In this case, the irradiation position control section 58 uses the turning drive device 11 or the traveling drive device for rotating the travel gantry 14 around the rotation axis 18 or the head swing device 15 preferentially prior to the couch drive device 42. Such movement reduces the load of moving the patient 43, which is preferable.

The therapeutic radiation irradiation section 59, after the therapeutic radiation irradiation device 16 is moved by the irradiation position control section 58, irradiates the affected area with the therapeutic radiation 23 by using the therapeutic radiation irradiation device 16.

The radiotherapy device control apparatus 2 can also be formed of a plurality of computers connected together so as to be capable of transmitting information bi-directionally to each other. In this case, the affected area position database 62, the three-dimensional data collection section 51, the treatment planning section 52, the affected area position table creation section 63, the imager position control section 53, the DRR image creation section 54, the transmitted image creation section 55, the reference image creation section 56, the affected area position control section 57, the output device control section 60, the motion collection section 61, the irradiation position control section 58, and the therapeutic radiation irradiation section 59 are each included in any of the plurality of computers. In this case, a plurality of users can perform radiotherapy related operation at separate places. For example, a computer for the user to create a treatment plan and a computer for another user to operate the radiotherapy device 3 can be provided separately.

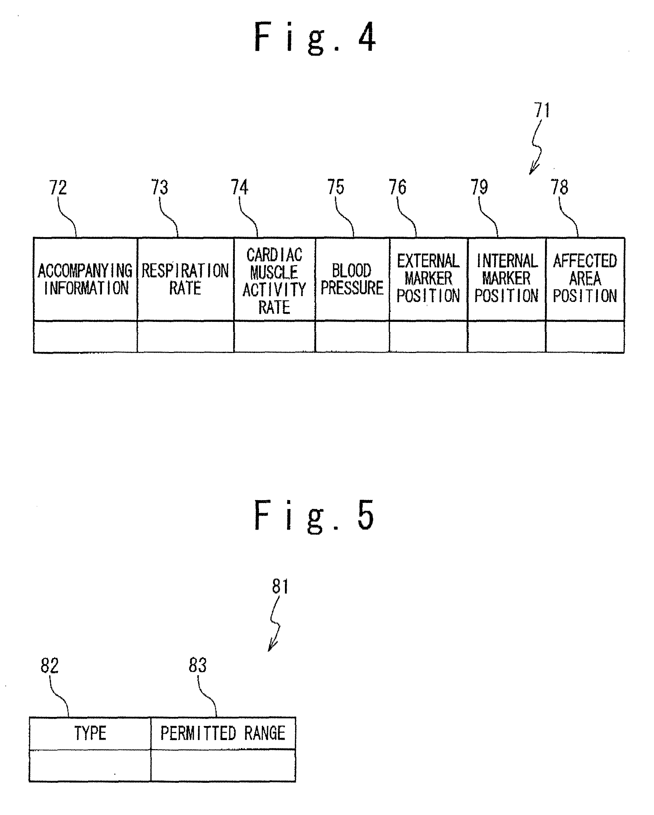

Fig. 4 is a view showing the affected area position table stored in the storage device by the affected area position database 62. The affected area position table 71 associates accompanying information 72 with motion information and associates the motion information with an affected area position 78. The motion information includes a respiration rate 73, a cardiac muscle activity rate 74, a blood pressure 75, an external marker position 76, and an internal marker position 79. The accompanying information 72 identifies the patient 43 and condition related to the motion of the patient 43, and indicates the name, sex, weight, date of birth, and carte number of the patient 43, name of a target affected area, photographing date, photographing angle, photographic X-ray condition, and chronological information. The chronological information indicates the condition and position of the affected area after photographing for a treatment plan or photographing before the photographing. The respiration rate 73 indicates, for a patient identified by the accompanying information 72, a combination of the respiration rate measured by the respirometer 6-1 and the average rate of change. The average rate of change indicates the average rate of change in the respiration rate at an interval between two time periods when the respiration rate is measured. The respiration rate 73 can further indicate a respiration phase calculated based on the respiration rate measured by the respirometer 6-1. The calculation method of the respiration phase is well known, and disclosed in, for example, Japanese Patent JP3326597B. The cardiac muscle activity rate 74 indicates, for a patient identified by the accompanying information 72, a combination of the activity rate measured by the electrocardiograph 6-2 and the average rate of change. The average rate of change indicates the average rate of change in the activity rate at an interval between two time periods when the activity rate is measured. The blood pressure 75 indicates, for a patient identified by the accompanying information 72, a combination of the blood pressure measured by the blood pressure meter 6-4 and the average rate of change. The average rate of change indicates the average rate of change in the blood pressure at an interval between two time periods when the blood pressure is measured. The external marker position 76 indicates a combination of the value indicating the position where a mark fitted to the body surface of the patient identified by the accompanying information 72 is displayed in a picture into which the patient was taken by the camera 6-5 and the average rate of change. The average rate of change indicates the average rate of change in the position at an interval between two time periods when the position is measured. The internal marker position 79 indicates a combination of the value indicating the position where a landmark inside the body of a patient identified by the accompanying information 72 is displayed in a picture into which the patient is taken by the imager system and the average rate of change in the value. The landmark is exemplified by bones (ribs), diaphragm, and bladder of the patient, and an object embedded in the patient so that it moves in conjunction with the affected area. The object can be detected by the imager system, and exemplified by a gold marker which is a sphere formed of gold. The average rate of change indicates the average rate of change in the position at an interval between two time periods when the position is measured. The affected area position 78 indicates the position where the affected area of the patient is arranged at a moment indicating the condition indicated by the respiration rate 73, the cardiac muscle activity rate 74, the blood pressure 75, the external marker position 76, and the internal marker position 79 for the patient identified by the accompanying information 72.

Specifically, the affected area position table creation section 63 creates the affected area position table 71 in association with three-dimensional data generated based on a plurality of transmitted images taken at a moment when a plurality of motion-related values are respectively measured by the respirometer 6-1, the electrocardiograph 6-2, the pulse meter 6-3, the blood pressure meter 6-4, the camera 6-5, and the imager systems (the diagnostic X-ray sources 24 and 25 and the sensor arrays 32 and 33, the therapeutic radiation irradiation device 16, the sensor array 31).

The irradiation position control section 58, referring to the affected area position table 71, calculates the position of affected area corresponding to the value collected by the motion collection section 61 and the average rate of change in the value, and moves the therapeutic radiation irradiation device 16 by using the head swing device 15 so that the therapeutic radiation 23 is transmitted through the three-dimensional position. The irradiation position control section 58 can also move the therapeutic radiation irradiation device 16 by using part of the condition indicated by the respiration rate 73, the cardiac muscle activity rate 74, the blood pressure 75, the external marker position 76, and the internal marker position 79. Further, the irradiation position control section 58 can also move the therapeutic radiation irradiation device 16 by using only the values measured by the motion detector 4 or can also move the therapeutic radiation irradiation device 16 by using only the average rate of change in the values.

The irradiation position control section 58 further controls the therapeutic radiation irradiation device 16 based on a permitted range table created by the user. Fig. 5 is a view showing the permitted range table 81. The permitted range table 81 associates a type 82 with a permitted range 83. The type 82 identifies the type of the value detected by the motion detector 4 and indicates any of the respiration rate and the average rate of change in the respiration rate, the activity rate and the average rate of change in the activity rate, the pulse and the average rate of change in the pulse, the blood pressure and the average rate of change in the blood pressure, the value indicating the position of a mark attached to the body surface and the average rate of change in the value. The permitted range 83 indicates the possible range of values identified by the types 82.

In this case, the irradiation position control section 58, referring to the permitted range table 81, stops radiation of the therapeutic radiation 23 by the therapeutic radiation irradiation device 16 when the value collected by the motion collection section 61 is out of the permitted range, and stops the radiation of the therapeutic radiation 23 by the therapeutic radiation irradiation device 16 when the average rate of change in the value collected by the motion collection section 61 is out of the permitted range.

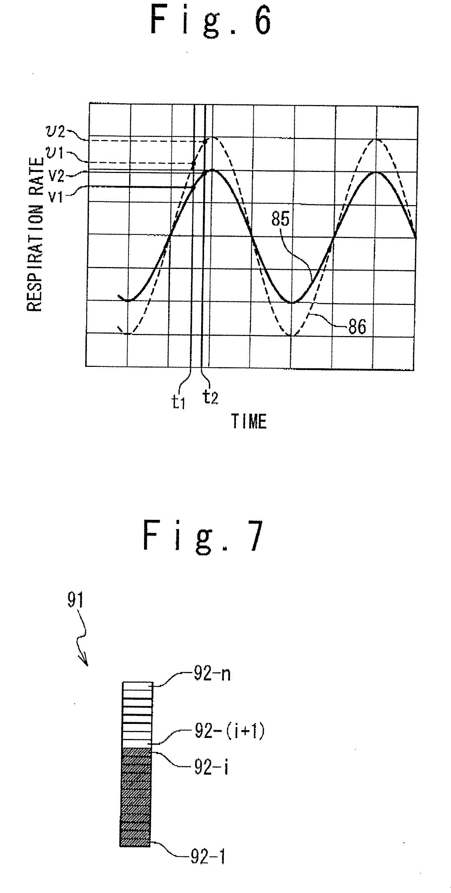

Fig. 6 is a graph showing changes in the respiration rate measured by the respirometer 6-1. The vertical axis indicates the respiration rate, and the horizontal axis indicates the time. The change 85 indicates the respiration rate when normal respiration is made, and the change 86 indicates the respiration rate when normal respiration is made. The changes 85 and 86 periodically change with time. The respirometer 6-1 intermittently measures the respiration rate. That is, when measuring the respiration rate at a time t1, the respirometer 6-1 measures the respiration rate at a time t2 after passage of a predetermined time from the time t1. The change 85 indicates the respiration rate V1 at the time t1 and the respiration rate V2 at the time t2. The change 86 indicates the respiration rate v1 at the time t1 and the respiration rate v2 at the time t2. In this case, the average rate of change in the respiration rate at the time t2 for the change 85 is expressed by the following formula:

$$(V2 - V1) / (t2-t1).$$

The average rate of change in the respiration rate at the time t2 for the change 86 is expressed by the following formula:

$$(v2-v1) / (t2-t1).$$

=

The affected area of the patient 43 moves to not only position corresponding to the respiration quantity but also position corresponding to such average rate of change. Moving the therapeutic radiation irradiation device 16 by the irradiation position control section 58 additionally based on the average rate of change is preferable in that the affected area of the patient 43 can be more reliably irradiated.

Fig. 7 is a view showing a screen displayed in the output device 7. The screen displays an indicator 91 of a rectangular shape. The indicator 91 is divided into a plurality of indicators 92-1 to 92-n (n = 2, 3, 4, ...) of a rectangular shape which are congruent with one another. The plurality of indicators 92-1 to 92-n are arranged in a row in the longitudinal direction of the indicator 91, filling the indicator 91 without leaving any clearance therein. The plurality of indicators 92-1 to 92-n correspond to respective possible ranges of respiration rate values, which ranges do not overlap with one another. In this case, when the range including the respiration rate requested to the patient 43 corresponds to one indicator 92-i (i = 1, 2, 3, ..., n), the output device control section 60 displays the indicators 92-1 to 92-i in one color while displaying the indicators 92-(i+1) to 92-n in one different color. Such display can show the periodical respiration timing to the patient 43, which is preferable.

The embodiment of a radiation irradiation method according to the present invention are executed by the radiotherapy system 1, and includes operation of creating a treatment plan, operation of creating an affected area position table, and operation of performing treatment.

In the operation of creating a treatment plan, the user first gathers three-dimensional data of an affected area of the patient 43 and a portion at the periphery of the affected area by using the CT 5, and stores the three-dimensional data in the storage device in association with identification information of the patient 43. The radiotherapy device control apparatus 2, based on the three-dimensional data generated by the CT 5, generates an image indicating the affected area of the patient 43 and organs at the periphery of the affected area. The user looks through the image by using the radiotherapy device control apparatus 2, and identifies the affected area position. The user, based on the image, further creates a treatment plan by using the treatment planning section 52, and inputs the treatment plan to the radiotherapy device control apparatus 2. The treatment plan indicates irradiation angles at which the affected area of the patient 43 is irradiated with the therapeutic radiation 23, and the dosage and property of the therapeutic radiation 23 irradiated at each of the irradiation angles. The treatment plan further indicates imaging angles at which the diagnostic X-rays 35 and 36 are irradiated upon the irradiation of the therapeutic radiation 23 at various irradiation angles. The imaging angles are calculated so that the transmitted images taken by transmitting the diagnostic X-rays 35 and 36 through the patient 43 display the affected area of the patient 43 more precisely. The radiotherapy device control apparatus 2 stores the treatment plan in the storage device in association with the identification information of the patient 43.

In the operation of creating the affected area position table, the user gathers three-dimensional data of an affected area of the patient 43 and a portion at the periphery of the affected area by using the CT 5 while measuring the motion of the patient 43 by using the motion detector 4. The radiotherapy device control apparatus 2, based on the values measured by the motion detector 4 and the three-dimensional data measured by the CT 5, creates the affected area position table 71. The user further gathers, by using the imager system of the radiotherapy device 3, the three-dimensional data of the affected area of the patient 43 and the portion at periphery of the affected area while storing the position where a landmark of the patient 43 is displayed in a transmitted image. When the gold marker is used as the landmark, after the gold marker is embedded in a portion moving in conjunction with the affected area of the patient 43, the user further gathers the three-dimensional data of the affected area of the patient 43 and the portion at periphery of the affected area while storing the position where the gold marker of the patient 43 is displayed in a transmitted image. The radiotherapy device control apparatus 2, based on the position where the landmark is displayed and the three-dimensional data of the affected area, creates the affected area position table 71.

In the operation of performing treatment, the user first fixes the patient 43 to the couch 41 of the radiotherapy device 3 in the same posture as when three-dimensional data is gathered by the CT 5 or the imager system of the radiotherapy device 3. The radiotherapy device control apparatus 2, based on the three-dimensional data collected in the operation of creating a treatment plan, calculates the DRR image. The DRR image indicates a transmitted image taken when the diagnostic X-rays 35 and 36 are irradiated at the imaging angles indicated by the treatment plan. The user, by using the radiotherapy device control apparatus 2, adds a mark to position in the DRR image where a characteristic point of the patient 43 is displayed so that the characteristic point displayed in the DRR image can be recognized.

Next, the radiotherapy device control apparatus 2 executes operation of position adjustment of the patient 43. Specifically, the radiotherapy device control apparatus 2 controls the radiation source drive device 37 to move the diagnostic X-ray source 24 so that the diagnostic X-rays 35 are irradiated to the patient 43 at the imaging angle indicated by the treatment plan, and controls the sensor array drive device 27 to move the sensor array 32 so that the transmitted image obtained by the diagnostic X-rays 35 mainly displays the affected area of the patient 43 in the center. The radiotherapy device control apparatus 2 further controls the radiation source drive device 38 to move the diagnostic X-ray source 25 so that the diagnostic X-rays 36 are irradiated to the patient 43 at the imaging angle indicated by the treatment plan, and controls the sensor array drive device 28 to move the sensor array 33 so that the transmitted image obtained by the diagnostic X-rays 36 mainly displays the affected area of the patient 43 in the center. The radiotherapy device control apparatus 2 further controls the turning drive device 11 or the traveling drive device to move the therapeutic radiation irradiation device 16 so that the therapeutic radiation 23 is irradiated to the patient 43 at the imaging angle indicated by the treatment plan, and controls the sensor array drive device 26 to move the sensor array 31 so that the transmitted image obtained by the therapeutic radiation 23 mainly displays the affected area of the patient 43.

The radiotherapy device control apparatus 2 radiates the diagnostic X-rays 35 by using the diagnostic X-ray source 24 and takes a transmitted image of the patient 43 generated by using the sensor array 32 based on the diagnostic X-rays 35. The radiotherapy device control apparatus 2 further radiates the diagnostic X-rays 36 by using the diagnostic X-ray source 25 and takes a transmitted image of the patient 43 generated by using the sensor array 33 based on the diagnostic X-rays 36. The radiotherapy device control apparatus 2 further radiates the therapeutic radiation 23 by using the therapeutic radiation irradiation device 16 and takes a transmitted image of the patient 43 generated by using the sensor array 31 based on the therapeutic radiation 23. The radiotherapy device control apparatus 2 compares the transmitted image with the DRR image to judge whether or not the affected area of the patient 43 is to be irradiated with the therapeutic radiation 23. The radiotherapy device control apparatus 2 further compares the transmitted image with the DRR image to calculate couch position of the couch 41. For example, the radiotherapy device control apparatus 2, based on a difference between the position of a characteristic point displayed in the transmitted image and the position of a characteristic point displayed in the DDR image, calculates the direction and distance in and by which the patient 43 is to be moved, and moves the couch 41 based on the direction and the distance. Or, the user controls the couch drive device 42 to move the patient 43 by using the radiotherapy device control apparatus 2 while viewing the display so that the taken transmitted image approximately matches with the DRR image. The radiotherapy device control apparatus 2 stores the taken image in the storage device in association with the identification information of the patient 43.

Next, the radiotherapy device control apparatus 2 repeatedly executes a tracking operation and an irradiation operation. In the tracking operation, the radiotherapy device control apparatus 2, by using the output device 7, directs the patient 43 to make predetermined respiration. The radiotherapy device control apparatus 2 further calculates the affected area position based on the values measured by the motion detector 4 and the position of a landmark detected by the imager system of the radiotherapy device 3. For example, the radiotherapy device control apparatus 2, referring to the affected area position table 71, calculates the affected area position corresponding to the values measured by the motion detector 4 and calculates the affected area position corresponding to the position of the landmark detected by the imager system of the radiotherapy device3. The radiotherapy device control apparatus 2 further judges whether or not the affected area position calculated based on the values measured by the motion detector 4 largely differs from the affected area position calculated based on the position of the landmark detected by the imager system. When the difference between the two affected area positions is small, the radiotherapy device control apparatus 2 moves the therapeutic radiation irradiation device 16 by using the head swing device 15 so that the therapeutic radiation 23 is transmitted through the affected area position. When the difference between the two affected area positions is large, the radiotherapy device control apparatus 2 moves the therapeutic radiation irradiation device 16 by using the head swing device 15 so that the therapeutic radiation 23 is transmitted through the affected area position corresponding to the position of the landmark detected by the imager system of the radiotherapy device 3. Such the tracking operation permits the calculation of the affected area position even when the imager system of the radiotherapy device 3 faces difficulty in directly detecting the affected area of the patient43. The correspondence between the values measured by the motion detector 4 and the affected area position changes with time, while the correspondence between the position of the landmark (gold marker in particular) detected by the imager system of the radiotherapy device 3 and the affected area position changes less. Such the tracking operation further permits preventing the calculated affected area position from becoming inappropriate due to chronological change in this correspondence.

Or, when the difference between the two affected area positions is large, the radiotherapy device control apparatus 2 stops the operation of performing treatment. Such the tracking operation further permits preventing a portion other than the affected area of the patient 43 from being irradiated in the event of breakdown, malfunction, or the like occurring in either the motion detector 4 or the imager system of the radiotherapy the device 3, therefore, such the tracking operation is preferable.

In this case, the radiotherapy device control apparatus 2 takes a transmitted image by a plurality of imager systems in frequency lower than frequency of measurements made by the motion detector 4, calculates three-dimensional affected area position based on the position where the affected area is displayed in the transmitted image, and moves the therapeutic radiation irradiation device 16 by using the head swing device 15 so that the therapeutic radiation 23 is transmitted through the three-dimensional position. With such irradiation of the diagnostic X-rays, the exposed dose for the patient 43 can be reduced.

The radiotherapy device control apparatus 2, referring to the affected area position table 71, can calculate the affected area position corresponding to the values measured by the motion detector 4 and to the position of the landmark detected by the imager system of the radiotherapy device 3.

In the irradiation operation, the radiotherapy device control apparatus 2 irradiates the therapeutic radiation 23 to the affected area by using the therapeutic radiation irradiation device 16 immediately after the therapeutic radiation irradiation device 16 is moved by the tracking operation. The radiotherapy device control apparatus 2, referring to the permitted range table 81, further stops radiation of the therapeutic radiation 23 by the therapeutic radiation irradiation device 16 when the value measured by the motion detector 4 is out of the permitted range and stops the radiation of the therapeutic radiation 23 by the therapeutic radiation irradiation device 16 when the average rate of change in the value is out of the permitted range. With such the tracking operation and the irradiation operation, even when the affected area is hard to detect by the imager system, the radiotherapy device 3 can identify the affected area with high accuracy, can more reliably irradiate only the affected area moving due to respiration or the like, and, as a result, can performs treatment with the higher accuracy.

The radiotherapy device control apparatus 2 can also calculate the affected area position by using the values measured by the motion detector 4 without using the position of the landmark detected by the imager system of the radiotherapy device 3 in the tracking operation. With such the radiation irradiation method, the accuracy deteriorates, but only the affected area moving due to respiration or the like can be irradiated and the dosage of X-rays irradiated to the patient 43 can be reduced, and as a result, treatment can be performed with higher accuracy. When a gold mark is used as the landmark, embedding the gold marker is the patient 43 is invasive processing, which imposes burden on the patient 43. That is, with the radiation irradiation method not using the gold marker, the burden imposed on the patient 43 due to the invasion can be reduced.

The radiotherapy device control apparatus 2 can also calculate the affected area position by using the position of the gold marker detected by the imager system of the radiotherapy device 3 without using the values measured by the motion detector 4 in the tracking operation. The radiotherapy device 3 cannot reduce the dosage of X-rays irradiated to the patient 43, but even when the affected area is hard to detect by the imager system, can identify the affected area with the high accuracy and can more reliably irradiate only the affected area moving due to respiration or the like, and as a result, can perform treatment with the higher accuracy.

A modified embodiment of the radiotherapy device control apparatus of the present invention further includes the three-dimensional data creation section 65 shown in Fig. 7 as a computer program. The three-dimensional data creation section 65, while rotating the travel gantry 14 around the rotation axis 18 by using the traveling drive device of the radiotherapy device 3, takes a transmitted image of the patient 43 by using the imager system of the radiotherapy device 3 and generates a plurality of transmitted images by transmitting X-rays through the human body from various directions. The three-dimensional data creation section 65 subjects the plurality of transmitted images to image processing by the computer to thereby generate images of cross sections of the human body and generate three-dimensional data indicating inner condition of the human body. Such image processing is well known.

A modified embodiment of the radiation irradiation method according to the present invention is executed by a radiotherapy system 1 to which such a radiotherapy device control apparatus is applied, with the operation of creating the treatment plan replaced with different operation and the operation of creating an affected area position table replaced with different operation in the aforementioned radiation irradiation method.

In the operation of creating the treatment plan, the user first fixes the patient 43 to the couch 41 of the radiotherapy device 3 and gathers three-dimensional data of an affected area of the patient 43 and a portion at the periphery of the affected area by using the imager systems of the radiotherapy device 3 (the therapeutic radiation irradiation device 16 and the sensor array 31, or the diagnostic X-ray source 24 and the sensor array 32). The radiotherapy device control apparatus 2, based on the three-dimensional data, generates an image indicating the affected area of the patient 43 and organs at the periphery of the affected area. The user looks through the image by using the radiotherapy device control apparatus 2 to identify the affected area position. The user further creates a treatment plan based on the image and inputs the treatment plan to the radiotherapy device control apparatus 2. The treatment plan indicates irradiation angles at which the therapeutic radiation 23 is irradiated to the affected area of the patient 43 and the dosage and condition of the therapeutic radiation 23 irradiated at the various irradiation angles. The treatment plan further indicates imaging angles at which the diagnostic X-rays 35 and 36 are irradiated upon the irradiation of the therapeutic radiation 23 at the various irradiation angles.

In the operation of creating an affected area position table, while measuring the motion of the patient 43 by using the motion detector 4, the user gathers three-dimensional data of the affected area of the patient 43 and the portion at the periphery of the affected area by using the imager systems of the radiotherapy device 3 (the therapeutic radiation irradiation device 16 and the sensor array 31, or the diagnostic X-ray source 24 and the sensor array 32). The radiotherapy device control apparatus 2, based on the values measured by the motion detector 4 and three-dimensional data measured by the imager systems of the radiotherapy device 3, creates the affected area position table 71.

With such the radiation irradiation method, the radiotherapy system 1 needs not to be provided with the CT 5 and can be reduced in dimension, and thus can be installed in smaller space and manufactured at lower costs.

In another modified embodiment of the radiation irradiation method according to the present invention, the operation of performing treatment in the embodiment already described is replaced with different operation.

In the operation of performing treatment, the user first fixes the patient 43 to the couch 41 of the radiotherapy device 3 in the same posture as when three-dimensional data is gathered by the CT 5. The radiotherapy device control apparatus 2, based on the three-dimensional data collected in the operation of creating a treatment plan, calculates the DRR image. The DRR image indicates transmitted images taken when the diagnostic X-rays 35 and 36 are irradiated at the imaging angles indicated by the treatment plan. The user, by using the radiotherapy device control apparatus 2, adds a mark to position in the DRR image where a characteristic point of the patient 43 is displayed so that the characteristic point displayed in the DRR image can be recognized.

The radiotherapy device control apparatus 2 controls the radiation source drive device 37 to move the diagnostic X-ray source 24 so that the diagnostic X-rays 35 are irradiated to the patient 43 at the imaging angle indicated by the treatment plan, and controls the sensor array drive device 27 to move the sensor array 32 so that the transmitted image obtained by the diagnostic X-rays 35 mainly displays the affected area of the patient 43 in the center. The radiotherapy device control apparatus 2 further controls the radiation source drive device 38 to move the diagnostic X-ray source 25 so that the diagnostic X-rays 36 are irradiated to the patient 43 at the imaging angle indicated by the treatment plan, and controls the sensor array drive device 28 to move the sensor array 33 so that the transmitted image obtained by the diagnostic X-rays 36 mainly displays the affected area of the patient 43 in the center.

The radiotherapy device control apparatus 2 radiates the diagnostic X-rays 35 by using the diagnostic X-ray source 24 and takes a transmitted image of the patient 43 generated by using the sensor array 32 based on the diagnostic X-rays 35. The radiotherapy device control apparatus 2 further radiates the diagnostic X-rays 36 by using the diagnostic X-ray source 25 and takes a transmitted image of the patient 43 generated by using the sensor array 33 based on the diagnostic X-rays 36. The radiotherapy device control apparatus 2 further radiates the therapeutic radiation 23 by using the therapeutic radiation irradiation device 16 and takes a transmitted image of the patient 43 generated by using the sensor array 31 based on the therapeutic radiation 23. The radiotherapy device control apparatus 2 controls the couch drive device 42 to move the patient 43 so that the taken transmitted image approximately matches with the DRR image.

Next, the radiotherapy device control apparatus 2 displays on the output device 7 a screen indicating the respiration rate requested to the patient 43, instructing the patient 43 to do so. The radiotherapy device control apparatus 2, referring the affected area position table 71, further calculates the affected area position corresponding to the values measured by the motion detector 4 and calculates the average rate of change measured by the motion detector 4. The radiotherapy device control apparatus 2 moves the therapeutic radiation irradiation device 16 by using the head swing device 15 and irradiates the therapeutic radiation 23 to the affected area by using the therapeutic radiation irradiation device 16 so that the therapeutic radiation 23 is transmitted through the affected area position during the period when the average rate of change is included in a predetermined range (for example, near zero).

With such operation, although the operation required more time than the embodiment already described, the therapeutic radiation 23 is irradiated only when the motion is small, the radiotherapy device 3 can more reliably irradiate only the affected area moving due to respiration or the like, and the radiation dosage for the patient 43 can be reduced, and as a result, treatment can be performed with higher accuracy.

It is apparent that the present invention is not limited to the above embodiment, that may be modified and changed without departing from the scope of the invention.

## Claims

1. A radiotherapy device control apparatus comprising:

an affected area position database (62);
a motion collection section (61); and
an irradiation position control section (58),

wherein said radiotherapy device control apparatus (2) controls a radiotherapy device (3),
wherein said radiotherapy device (3) includes:

a therapeutic radiation irradiation device (16) which irradiates a part of a subject with therapeutic radiation,
a motion detector (4) which detects motion of said subject, and
a drive device (11, 15, 42) which moves said therapeutic radiation irradiation device (16) with respect to said subject,

wherein said affected area position database (62) associates a position set with a motion related information set related to said motion,
wherein said motion collection section (61) collects said motion from said motion detector (4), and
wherein said irradiation position control section (58) moves said therapeutic radiation irradiation device (16) by said drive device (11, 15, 42) such that said therapeutic radiation is radiated to a position in said position set corresponding to motion related information of said motion.

2. The radiotherapy device control apparatus according to claim 1, wherein said motion related information set is a motion set, and said motion related information is said motion.

3. The radiotherapy device control apparatus according to claim 1 or 2, wherein said motion indicates a landmark position at which a landmark arranged in said subject is displayed in a transmitted image taken by an imager of said radiotherapy device (3) using radiation transmitted through said subject.

4. The radiotherapy device control apparatus according to any one of claims 1 to 3, wherein said affected area position database (62) further associates a set of an average rate of change with said position set, and wherein said irradiation position control section (58) moves said therapeutic radiation irradiation device (16) by said drive device (11, 15, 42) such that said therapeutic radiation is radiated to a position in said position set further corresponding to an average rate of change of said motion.

5. The radiotherapy device control apparatus according to anyone of claims 1 to 4, wherein said irradiationposition control section (58) makes said therapeutic radiation irradiation device (16) stop radiating said therapeutic radiation if said motion is not included in a predetermined range.

6. The radiotherapy device control apparatus according to any one of claims 1 to 5, further comprising:

an affected area position table creation section (63) which creates said affected area position database (62) based on variation of a three-dimensional data of said subject created by a three-dimensional imaging device provided in addition to said radiotherapy device and variation of a motion detected by said motion detector (4) .

7. The radiotherapy device control apparatus according to any one of claims 1 to 5, further comprising:

an affected area position table creation section (63) which creates said affected area position database (62) based on variation of a three-dimensional data of said subject created by using a transmitted image taken by an imager of said radiotherapy device (3) using transmitted radiation transmitted through said subject and variation of a motion detected by said motion detector (4).

8. The radiotherapy device control apparatus according to claim 7, wherein said transmitted radiation is generated by said therapeutic radiation being transmitted through said subject.

9. The radiotherapy device control apparatus according to claim 1, wherein said motion related information set is a set of an average rate of change, and said motion related information is an average rate of change of said motion.

10. A radiotherapy device control apparatus comprising:

a motion collection section (61); and
a therapeutic radiation irradiation section (59),

wherein said radiotherapy device control apparatus (2) controls a radiotherapy device (3),
wherein said radiotherapy device (3) includes:

a therapeutic radiation irradiation device (16) which radiates therapeutic radiation, and
a motion detector (4) which detects motion of said subject,

wherein said motion collection section (61) collects said motion from said motion detector (4), and wherein said therapeutic radiation irradiation section (59) makes said therapeutic radiation irradiation device (16) radiate said therapeutic radiation when said average rate of change is included in a predetermined range, and makes said therapeutic radiation irradiation device (16) stop radiating said therapeutic radiation when said average rate of change is not included in said predetermined range.

11. A radiotherapy system comprising:

a radiotherapy device control apparatus according to any one of claims 1 to 10; and
a radiotherapy device (3).

12. A radiation irradiation method using a radiotherapy device (3), wherein said radiotherapy device (3) includes:

a therapeutic radiation irradiation device (16) which irradiates a part of a subject with therapeutic radiation,
a motion detector (4) which detects motion of said subject, and
a drive device (11, 15, 42) which moves said therapeutic radiation irradiation device (16) with respect to said subject,

said radiation irradiation method comprising:

(a) collecting said motion from said motion detector (4); and
(b) moving said therapeutic radiation irradiation device (16) by said drive device (11, 15 , 42) with reference to an affected area position database (62) associating a position set with a motion related information set related to said motion, such that said therapeutic radiation is radiated to a position in said position set corresponding to motion related information of said motion.

13. The radiation irradiation method according to claim 12, wherein said motion related information set is a motion set, and said motion related information is said motion.

14. The radiation irradiation method according to claim 12 or 13, wherein said motion indicates a landmark position at which a landmark arranged in said subject is displayed in a transmitted image taken by an imager of said radiotherapy device (3) using radiation transmitted through said subject.

15. The radiation irradiation method according to any one of claims 12 to 14, wherein said affected area position database (62) further associates a set of an average rate of change with said position set, and wherein said position further corresponds to an average rate of change of said motion.

16. The radiation irradiation method according to any one of claims 12 to 15, further comprising:

(c) making said therapeutic radiation irradiation device (16) stop radiating said therapeutic radiation if said motion is not included in a predetermined range.

17. The radiation irradiation method according to any one of claims 12 to 16, further comprising:

(e) creating said affected area position database (62) based on variation of a three-dimensional data of said subject created by a three-dimensional imaging device provided in addition to said radiotherapy device and variation of a motion detected by said motion detector (4) .

18. The radiation irradiation method according to any one of claims 12 to 17, further comprising:

(f) creating said affected area position database (62) based on variation of a three-dimensional data of said subject created by using a transmitted image taken by an imager of said radiotherapy device (3) using transmitted radiation transmitted through said subject and variation of a motion detected by said motion detector (4).

19. The radiation irradiation method according to claim 18, wherein said transmitted radiation is generated by said therapeutic radiation being transmitted through said subject.

20. The radiation irradiation method according to claim 12, wherein said motion related information set is a set of an average rate of change, and said motion related information is an average rate of change of said motion.

21. A radiation irradiation method using a radiotherapy device (3), wherein said radiotherapy device (3) includes:

a therapeutic radiation irradiation device (16) which radiates therapeutic radiation, and a motion detector (4) which detects motion of said subject,

said radiation irradiation method comprising:

(g) collecting said motion from said motion detector (4); and
(h) making said therapeutic radiation irradiation device (16) radiate said therapeutic radiation when said average rate of change is included in a predetermined range; and
(i) making said therapeutic radiation irradiation device (16) stop radiating said therapeutic radiation when said average rate of change is not included in said predetermined range.

22. Computer program product with program code means for carrying out all steps according to any one of claims 12 to 21 if the program runs on a computer.

23. Computer program product with program code means according to claim 22 which are stored on a storage means which can be read by the computer.

# F i g . 1

# Fig. 2

# F i g . 3

2

```
┌──────────────────────────────────────────────────┐
│         RADIOTHERAPY DEVICE CONTROL APPARATUS      │
│  ┌──────────────────────────────────────────────┐ │
│  │        AFFECTED AREA POSITION DATABASE         │─┼─62
│  └──────────────────────────────────────────────┘ │
│  ┌──────────────────────────────────────────────┐ │
│  │      THREE-DIMENSIONAL DATA COLLECTION SECTION │─┼─51
│  └──────────────────────────────────────────────┘ │
│  ┌──────────────────────────────────────────────┐ │
│  │            TREATMENT PLANNING SECTION          │─┼─52
│  └──────────────────────────────────────────────┘ │
│  ┌──────────────────────────────────────────────┐ │
│  │  AFFECTED AREA POSITION TABLE CREATION SECTION │─┼─63
│  └──────────────────────────────────────────────┘ │
│  ┌──────────────────────────────────────────────┐ │
│  │         IMAGER POSITION CONTROL SECTION        │─┼─53
│  └──────────────────────────────────────────────┘ │
│  ┌──────────────────────────────────────────────┐ │
│  │           DRR IMAGE CREATION SECTION           │─┼─54
│  └──────────────────────────────────────────────┘ │
│  ┌──────────────────────────────────────────────┐ │
│  │       TRANSMITTED IMAGE CREATION SECTION       │─┼─55
│  └──────────────────────────────────────────────┘ │
│  ┌──────────────────────────────────────────────┐ │
│  │        REFERENCE IMAGE CREATION SECTION        │─┼─56
│  └──────────────────────────────────────────────┘ │
│  ┌──────────────────────────────────────────────┐ │
│  │      AFFECTED AREA POSITION CONTROL SECTION    │─┼─57
│  └──────────────────────────────────────────────┘ │
│  ┌──────────────────────────────────────────────┐ │
│  │          OUTPUT DEVICE CONTROL SECTION         │─┼─60
│  └──────────────────────────────────────────────┘ │
│  ┌──────────────────────────────────────────────┐ │
│  │            MOTION COLLECTION SECTION           │─┼─61
│  └──────────────────────────────────────────────┘ │
│  ┌──────────────────────────────────────────────┐ │
│  │        IRRADIATION POSITION CONTROL SECTION    │─┼─58
│  └──────────────────────────────────────────────┘ │
│  ┌──────────────────────────────────────────────┐ │
│  │    THERAPEUTIC RADIATION IRRADIATION SECTION   │─┼─59
│  └──────────────────────────────────────────────┘ │
│  ┌──────────────────────────────────────────────┐ │
│  │     THREE-DIMENSIONAL DATA CREATION SECTION    │─┼─65
│  └──────────────────────────────────────────────┘ │
└──────────────────────────────────────────────────┘
```

# Fig. 4

| ACCOMPANYING INFORMATION | RESPIRATION RATE | CARDIAC MUSCLE ACTIVITY RATE | BLOOD PRESSURE | EXTERNAL MARKER POSITION | INTERNAL MARKER POSITION | AFFECTED AREA POSITION |
|---|---|---|---|---|---|---|
| | | | | | | |

# Fig. 5

| TYPE | PERMITTED RANGE |
|---|---|
| | |

# F i g . 6

# F i g . 7

**European Patent Office**

## PARTIAL EUROPEAN SEARCH REPORT

which under Rule 45 of the European Patent Convention shall be considered, for the purposes of subsequent proceedings, as the European search report

Application Number

EP 07 10 2488

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/54689 A (ACCURAY INC [US])<br>21 September 2000 (2000-09-21)<br>* abstract * | 1-3,5-7,<br>11 | INV.<br>A61N5/10 |
| Y | * page 3, line 9 - page 4, line 15 *<br>* page 6, line 14 - page 21, line 20 *<br>----- | 4,8-10 | |
| X | WO 02/19908 A (ACCURAY INC [US])<br>14 March 2002 (2002-03-14)<br>* abstract *<br>* page 3, line 8 - page 4, line 22 *<br>* page 7, line 17 - page 28, line 2 *<br>----- | 1-3,5-7,<br>11 | |
| X | WO 2006/005021 A (ACCURAY INC [US];<br>SARACEN MICHAEL [US])<br>12 January 2006 (2006-01-12)<br>* abstract *<br>* paragraph [0032] - paragraph [0037] *<br>* paragraph [0043] - paragraph [0048] *<br>* paragraph [0052] *<br>* paragraph [0060] - paragraph [0066] *<br>* paragraph [0073] *<br>----- | 1-3,6,7,<br>11 | |
| | -/-- | | TECHNICAL FIELDS SEARCHED (IPC)<br><br>A61N |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 18 June 2007 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**PARTIAL EUROPEAN SEARCH REPORT**

Application Number

EP 07 10 2488

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| Y | EP 0 812 605 A2 (WISCONSIN ALUMNI RES FOUND [US]) 17 December 1997 (1997-12-17)<br>* abstract *<br>* column 4, line 16 - line 57 *<br>----- | 8 | |
| Y | WO 2004/112607 A (WISCONSIN ALUMNI RES FOUND [US]; ZHANG TIEZHI [US]; KELLER HARALD [US]) 29 December 2004 (2004-12-29)<br>* abstract *<br>* paragraph [0063] *<br>----- | 4,9,10 | |
| P,X | WO 2006/039586 A (ACCURAY INC [US]; SAYEH SOHAIL [US]) 13 April 2006 (2006-04-13)<br>* the whole document *<br>----- | 1 | |
| P,X | WO 2006/039009 A (ACCURAY INC [US]; THOMSON EUAN [US]; DOOLEY JOHN ROBINSON [US]; KUDUVA) 13 April 2006 (2006-04-13)<br>* the whole document *<br>----- | 1 | TECHNICAL FIELDS SEARCHED (IPC) |
| A | WO 99/27839 A2 (COSMAN ERIC R [US]) 10 June 1999 (1999-06-10)<br>* abstract *<br>* page 4, line 21 - page 14, line 22 *<br>----- | 1 | |
| A | US 2002/077545 A1 (TAKAHASHI SHUICHI [JP] ET AL) 20 June 2002 (2002-06-20)<br>* paragraph [0029] - paragraph [0033] *<br>* paragraph [0043] - paragraph [0094] *<br>----- | 1 | |

EPO FORM 1503 03.82 (P04C10)

**INCOMPLETE SEARCH**
**SHEET C**

Application Number

EP 07 10 2488

```
Claim(s) not searched:
        12-23

Reason for the limitation of the search (non-patentable invention(s)):

Claims 12-21: Article 52 (4) EPC - Method for treatment of the human or
animal body by therapy
Claims 22 and 23: Article 84 EPC
```

# EP 1 832 313 A1

## ANNEX TO THE EUROPEAN SEARCH REPORT
## ON EUROPEAN PATENT APPLICATION NO.

EP 07 10 2488

This annex lists the patent family members relating to the patent documents cited in the above-mentioned European search report.
The members are as contained in the European Patent Office EDP file on
The European Patent Office is in no way liable for these particulars which are merely given for the purpose of information.

18-06-2007

| Patent document cited in search report | | Publication date | Patent family member(s) | | Publication date |
|---|---|---|---|---|---|
| WO 0054689 | A | 21-09-2000 | AT | 269035 T | 15-07-2004 |
| | | | AU | 3882700 A | 04-10-2000 |
| | | | DE | 60011607 D1 | 22-07-2004 |
| | | | DE | 60011607 T2 | 30-06-2005 |
| | | | EP | 1176919 A1 | 06-02-2002 |
| | | | JP | 2003523220 T | 05-08-2003 |
| | | | US | 6144875 A | 07-11-2000 |
| WO 0219908 | A | 14-03-2002 | AU | 9068801 A | 22-03-2002 |
| | | | US | 2003125622 A1 | 03-07-2003 |
| | | | US | 6501981 B1 | 31-12-2002 |
| WO 2006005021 | A | 12-01-2006 | EP | 1778358 A2 | 02-05-2007 |
| | | | US | 2006004281 A1 | 05-01-2006 |
| EP 0812605 | A2 | 17-12-1997 | CA | 2207539 A1 | 11-12-1997 |
| | | | DE | 69730269 D1 | 23-09-2004 |
| | | | DE | 69730269 T2 | 25-08-2005 |
| | | | JP | 10099456 A | 21-04-1998 |
| | | | US | 5673300 A | 30-09-1997 |
| WO 2004112607 | A | 29-12-2004 | EP | 1633246 A1 | 15-03-2006 |
| | | | US | 2004254492 A1 | 16-12-2004 |
| WO 2006039586 | A | 13-04-2006 | EP | 1793738 A2 | 13-06-2007 |
| | | | US | 2006074299 A1 | 06-04-2006 |
| | | | US | 2006074304 A1 | 06-04-2006 |
| WO 2006039009 | A | 13-04-2006 | EP | 1793734 A2 | 13-06-2007 |
| | | | US | 2006074292 A1 | 06-04-2006 |
| WO 9927839 | A2 | 10-06-1999 | AU | 1800999 A | 16-06-1999 |
| | | | CA | 2320230 A1 | 10-06-1999 |
| | | | DE | 69833881 T2 | 07-12-2006 |
| | | | EP | 1642545 A1 | 05-04-2006 |
| | | | EP | 1041918 A2 | 11-10-2000 |
| | | | ES | 2256974 T3 | 16-07-2006 |
| | | | US | 2002065461 A1 | 30-05-2002 |
| | | | US | 2002188194 A1 | 12-12-2002 |
| | | | US | 2004122311 A1 | 24-06-2004 |
| | | | US | 6405072 B1 | 11-06-2002 |
| US 2002077545 | A1 | 20-06-2002 | DE | 10147633 A1 | 04-07-2002 |
| | | | JP | 2002177406 A | 25-06-2002 |

EPO FORM P0459

For more details about this annex : see Official Journal of the European Patent Office, No. 12/82

27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 37467478 B
- JP 2004121406 A
- JP 3053389 B
- US 6307914 B
- JP 3432268 B
- JP 3326597 B

- JP H4507048 A
- WO 9011721 A
- JP 3394250 B
- WO 92006644 A
- JP H8511452 A
- WO 9428971 A